# EUROPEAN PATENT APPLICATION

(11) **EP 1 013 340 A2**
(43) Date of publication of application: **28.06.2000**
(21) Application number: 99123663.9
(22) Date of filing: 29.11.1999
(51) Int. Cl.: B01J 31/06, C07C 45/50, C07C 51/12, C07C 7/163

(54) **Polymer supported triphos compound and use thereof as catalyst**

(30) Priority: 23.12.1998 US 113868 P
(71) Applicant: Haldor Topsoe A/S, DK-2800 Lyngby (DK)
(72) Inventor: Schiodt, Niels Christian, 2700 Bronshoj (DK); Joensen, Finn, 2970 Horsholm (DK)

(57) **Abstract**

Material having the general chemical formula 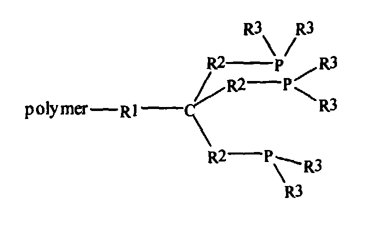 where R¹ is a linker and C(R²P(R³)₂)₃ group is a triphos moiety; and
wherein
R¹ is selected from branched and linear alkyl group and alkyl carbonyl group optionally substituted with functional groups comprising carbonyl, alkoxy, hydroxy, amino, nitro, carboxylic or sulfonic acid and esters or amides thereof;
R² are methylene (CH₂) or substituted methylene groups;
R³ are linear or branched alkyl or phenyl or substituted phenyl groups;
polymer is any polymer containing aromatic groups, comprising polystyrene, poly-vinyl pyridine, poly pyrrole, poly pyrrolidone or derivatives thereof.

## Description

The present invention relates to unknown polymeric materials with tripodal chelating tris (diphenylphosphinomethyl) methyl groups covalently bound to surface of a polymer. These polymeric materials are easily impregnated with transition metals, after which they may serve as immobilised molecular catalysts with respect to a variety of reactions.

It is well known that the role of a catalyst is to increase the rate and selectivity of a chemical reaction. Such catalyst may be heterogeneous (insoluble in the reaction medium) or homogeneous (soluble). Each kind has some advantages and some drawbacks. The homogeneous catalysts are often well defined transition metal complexes with a high selectivity, but the separation of the catalyst from the product imposes an inherent problem. Therefore, it is desirable to immobilise such homogeneous catalysts on an insoluble support.

The homogeneous catalysts are typically transition metal complexes consisting of a transition metal ion and some ligands. Phosphine ligands are among the most common, as e.g. in the Rhone-Poulenc hydroformylation process (B. Cornils and E. Wiebus chemtech 25, 33, 1995). Phosphine ligands are furthermore suitable for covalent immobilisation and phosphine-modified supports have been well known for years (G. O. Evans, C. U. Pittman Jr., R. McMillan, R. T. Beach and R. Jones J. Organomet. Chem. 67, 295, 1974; K. G. Allum, R. D. Hancock, I. V. Howell, R. C. Pitkethly and P. J. Robinson Jones J. Organomet. Chem. 87, 189, 1975).

During the past two decades, transition metal complexes of the tridentate phosphine ligand 1,1,1-tris (diphenylphosphinomethyl) ethane (commonly called triphos) have attracted increasing attention within the field of homogeneous catalysis. Thus, it has been reported that a rhodium complex with triphos is an active hydroformylation catalyst (C. Bianchini, A. Meli, M. Peruzzini, F. Vizza, P. Frediani and J. A. Ramirez Organometallics 9, 226, 1990), and a very thorough study of hydrodesulfurisation by rhodium, iridium and ruthenium complexes with triphos has been published (C. Bianchini and A. Meli in T. Weber et al (eds.) Transition Metal Sulphides, Kluwer Academic Publishers, 1998).

It has been found that by swelling material based on a polymer resin with covalently bound triphos-type groups in a solution containing suitable transition metal salts or complexes, the metal ions are captured by the resin-bound triphos ligands. Thus, the immobilised triphos complexes are catalytically active at different reactions.

### SUMMARY OF THE INVENTION

The invention provides materials, which generally are characterised by the following three parts:
polymer - linker - triphos
represented by the general chemical formula: 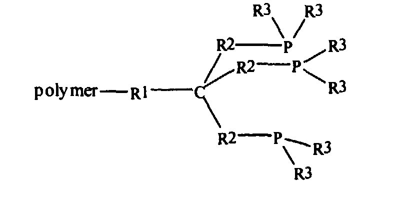 where R¹ is the linker and the C(R²P(R³)₂)₃ group is the triphos part.

R¹ is selected from branched and linear alkyl group and alkyl carbonyl group optionally substituted with functional groups comprising carbonyl, alkoxy, hydroxy, amino, nitro, carboxylic or sulfonic acid and esters or amides thereof.

R² are methylene (CH₂) or substituted methylene groups. R³ are linear or branched alkyl or phenyl or substituted phenyl groups.

The polymer may be any polymer containing aromatic groups, such as polystyrene, poly-vinyl pyridine, poly pyrrole, poly pyrrolidone or derivatives thereof, such as the said polymers substituted with one or more alkyl, formyl, alkyl carbonyl, alkoxy, hydroxy, amino, nitro or carboxylic or sulfonic acid and esters or amides thereof in the aromatic ring. The polymer may also be a copolymer, e.g. a grafted polymer or block-copolymer as long as aromatic groups are present for functionalisation with the triphos ligand moiety.

The linker-triphos groups are covalently bound to the phenyl groups of the polymer, thus providing immobilised sites for chelation of transition metal ions.

### Examples

### Example 1

### Preparation of polymer carrier.

Two polymers were used for the preparation of the materials described here. PEPS, which is polystyrene (PS) grafted on the surface of polyethylene (PE) was prepared as follows: Polyethylene grains (0.2-0.4 mm) were crossbound by irradiation in an electron accelerator facility with a dose of 298 kGy. The crossbound PE was then immersed in a solution of styrene in methanol (30% v/v styrene) and submitted to γ-irradiation from a ⁶⁰Co source with a dose of 25 kGy. The material was washed thoroughly with methanol and with dichloromethane. It was then dried in a vacuum oven at 40°C for at least 8 hours. The material thus obtained contains 34.2 % by weight polystyrene. The triphos-derived resin based on PEPS is called I (see the synthesis scheme below).

Using a commercial polymer such as PS crossbound with divinylbenzene could give rise to a material similar to I with respect to chemical properties, but with better thermal and mechanical properties.

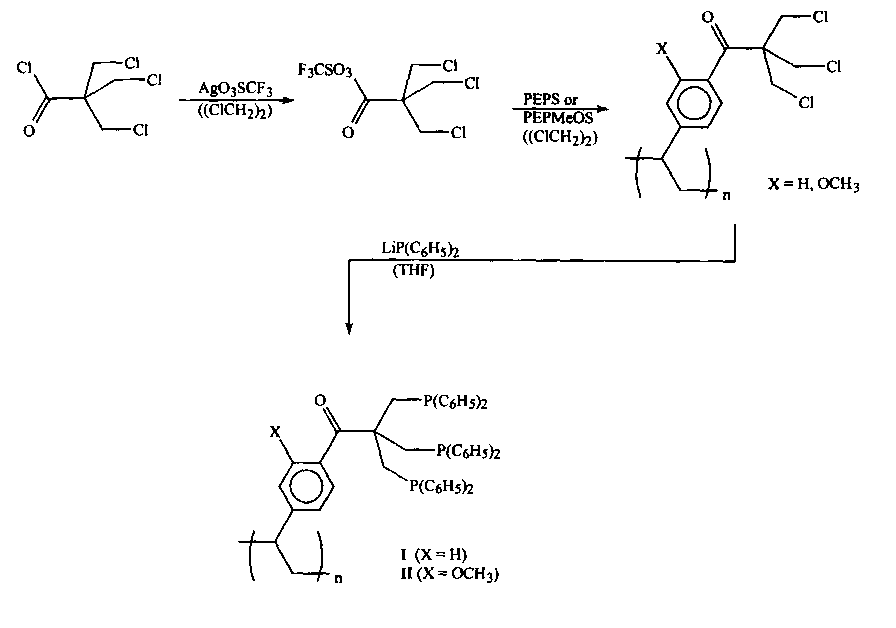

### Example 2

### Preparation of polymer carrier.

The other polymer PEPMeOS was prepared in exactly the same manner, but with 3-methoxystyrene instead of styrene. This gave rise to a material containing 35.6% poly(3-methoxy)styrene. The triphos-derived resin based on PEPMeOS is called II.

The synthesis of I and II were carried out as schematised below.

The synthesis of tris(chloromethyl)acetyl chloride ((ClCH₂)₃CCOCl) from pentaerythrithol is described in the literature (A. Mooradian and J. B. Cloke, J. Chem. Soc. 67, 942, 1945).

### Example 3

### Functionalisation of polymer carrier.

Tris(chloromethyl)acetyl chloride (ClCH₂)₃CCOCl (66.80 g; 0.30 mole) was dissolved in 1,2-dichloroethane (300 ml) at and stirred magnetically with an equimolar amount of solid silver trifluoromethanesuphonate (62.00 g; 0.24 mole) at room temperature for 24 hours in a dry atmosphere in the dark. This caused the formation of an almost quantitative precipitate of silver chloride, which was filtered off in a dry atmosphere and with dry equipment. The filtrate containing the mixed carboxylic acid trifluoromethanesulfonic acid anhydride was then used for the acylation of the polymer. The polymer (PEPS) in the form of small grains (100 g; equivalent to 34.2 g PS; 0.33 mole) were added to the filtrate. An additional 400 ml 1,2-dichloroethane were added and the temperature was raised to the boiling point of the solvent and kept there for 3 hours. After having cooled the reaction flask to room temperature, the polymer was washed with 1,2 dichloro-ethane and then within heptane and dried until constant weight was achieved.

The acylated polymer was converted to I by treatment with a solution of lithium diphenylphosphide in tetrahydrofuran (THF) prepared according to literature methods (G. Kordosky, B. R. Cook, J. Cloyd Jr. and D. W. Meek Inorg. Synth. Coll. Vol. XIV, 14, 1973). All operations involving diphenylphosphide anion as well as the product polymer I were conducted in an argon atmosphere to prevent oxidation. The best results were obtained by cooling the lithium diphenylphosphide solution (0.25 mole) to -78°C, adding the thoroughly dried polymer (containing approximately 0,075 mole Cl) in one portion and allowing the slurry (magnetically stirred) to warm up to room temperature within one hour and subsequent refluxing the slurry for 2 hours. Then the excess diphenylphosphide was destroyed by the addition of acetic acid (6 g) followed by the addition of 100 ml ethanol. The polymer was filtered off and washed with 250 ml of each of the following solvents: Ethanol, water, acetone (twice), benzene and light petroleum ether. The polymer was dried in a vacuum oven at 40°C.

### Preparation of Transition Metal Impregnated I and II

### Example 4A

### Preparation of rhodium impregnated I (I-Rh).

A stream of CO was led through 50 ml of toluene for 20 minutes at room temperature. While maintaining a slow stream of CO, Rh₂Cl₂(CO)₄ (1.5 g) were dissolved in the toluene. A sample of I (10 g) was added to the yellow solution, causing a vigorous evolution of CO (bubbles). Within 15 minutes the polymer had turned red while the colour of the solution had fainted. The slurry was stirred for a further 4 hours. The polymer was then filtered off, washed with toluene until the washings were completely colourles and then with petroleum ether. The polymer was dried overnight in a stream of CO. Weight: 10.80 g.

### Example 4B

### Preparation of rhodium impregnated II (II-Rh).

This material was prepared in the same way as I-Rh. From 8 g of II was obtained 8.75 g of II-Rh.

### Example 4C

### Preparation of ruthenium impregnated I (I-Ru).

Hydrated RuCl₃ (2 g) were suspended in acetonitrile (20 ml) and treated with neat trifluoromethane sulfonic acid (10 ml) and heated to reflux for two hours. The red solution was cooled to room temperature and filtered. The filtrate was diluted with 50 ml acetonitrile and I (10 g) was suspended therein. The suspension was left overnight with gentle stirring. The supernatant solution had changed in colour to bright orange, while the polymer had turned orange. The polymer was filtered off, washed with acetonitrile and dried in the air. Weight: 10.57 g.

### Example 4D

### Preparation of palladium impregnated I (I-Pd).

PdCl₂ (0.18 g) was dissolved in 100 ml boiling acetonitrile. The polymer I (2 g) was added together with 20 ml of toluene. The temperature was raised to the boiling point and the volume diminished by distillation. When the volume was approximately 25 ml, the slurry was filtered. The polymer (now red) was washed with boiling acetonitrile (25 ml), boiling toluene (25 ml) and once again with boiling acetonitrile (50 ml) and air dried. Weight: 2.10 g.

### Example 4E

### Preparation of platinum impregnated I (I-Pt).

PtI₂ (0.45 g) was refluxed with benzonitrile (50 ml) for 2 hours. The solution was cooled to room temperature and filtered. To the filtrate was added I (2 g) and the slurry was heated to reflux for 10 minutes and again cooled to room temperature. The polymer was filtered, washed with boiling acetonitrile (25 ml), boiling toluene (25 ml) and once again with hot acetonitrile (25 ml). The red material was dried in the air for several hours. Weight: 2.31 g.

### Examples of Catalytic Activity of the Metal Impregnated Materials

### Example 5

### Methanol carbonylation with I-Rh and II-Rh.

In a typical experiment, a laboratory scale reactor (150 ml) was loaded with methanol, methyl acetate, water, methyl iodide and I-Rh or II-Rh according to the table. The reactor contents were flushed with CO, the reactor was sealed, pressurised to 10 bar with CO, heated to the reaction temperature (185°C) and then connected to a CO-reservoir, increasing the pressure to 35 bar. The CO-reservoir allows for the reaction to be followed continuously, giving a precise rate measurement in terms of the rate of CO consumption. After 0.5-4 hours the autoclave was cooled and depressurised. By the impregnation procedure described above, some unbound rhodium becomes absorbed in the polymer. This was washed out by submitting the material to three consecutive catalytical runs after which of each the polymer was filtered off, washed with methanol and dried (total run time was in each case 10-14 hours). The Turn-Over-Frequencies (TOF) given in Table 1 as the number of moles CO consumed pr kilogram of catalyst per hour are recorded for this pretreated catalyst.

**Table 1**

| Catalyst | Methyl iodide | Methyl acetate | Acetic acid | Water | TOF (mol kg⁻¹ hr⁻¹) |
|---|---|---|---|---|---|
| 4.0 g I-Rh | 7.28 g | 20 g | 10 g | 6 g | 51 |
| 3.8 g I-Rh | 0.83 g | 20 g | 15 g | 6 g | 6 |
| 4.2 g II-Rh | 0.75 g | 20 g | 15 g | 6 g | 7 |

### Example 6

### Hydrodesulphurisation and hydrogenation with I-Rh(H) and I-Ru(H).

For hydrodesulfurisation, I-Rh was pretreated the following way: A reactor was loaded with 10 g I-Rh and 50 ml of toluene. The reactor was flushed with H₂, sealed, heated to 180°C, pressurised to 30 bar with H₂ and left for 20 hours. After cooling, the hydrogenated catalyst (I-Rh(H)) was filtered off and dried in a stream of hydrogen. I-Ru(H) was prepared likewise.

In a typical experiment, a plug-flow reactor was loaded with the catalyst, the system evacuated and refilled with hydrogen and heated to the relevant temperature in a steady flow of feed at the rate of 0.5 ml/min in a stream of H₂ at 250 ml/min and a pressure of 30 bar. The feed composition was 3% dibenzo-thiophene (DBT), 1.5% benzo-thiophene (BT) and 1% naphtalene (NAP) in n-heptane. By on-line gas chromatography, the concentration of substrate and conversion products were measured every second hour. The amount of catalyst and the conversions are to be found in Table 2.

**Table 2**

| Example No. | Catalyst type and amount | Temperature | DBT-conversion | BT-conversion | NAP-conversion |
|---|---|---|---|---|---|
| 1 | 0.30 g I-Rh(H) | 157°C | 0.0% | 4.6 % | 0.0% |
| 2 | 0.30 g I-RH(H) | 177°C | 0.0% | 6.9 % | 0.0% |
| 3 | 0.30 g I | 157°C | 0.0% | 0.0 % | 0.0% |
| 4 (initial rate) | 0.30 g I-Ru(H) | 158°C | 23.3% | 80.6% | 15.7% |
| 4 (after 12.8 hrs) | 0.30 g I-Ru(H) | 158°C | 9.3% | 26.6% | 4.4% |

### Example 7

### Hydroformylation of 1-heptene with I-Rh.

In a typical experiment, a laboratory scale reactor (150 ml) were loaded with toluene (20 g), 1-heptene (2 g ) and I-Rh according to the Table. The mixture was flushed with CO, the reactor was sealed and heated to the reaction temperature (100°C). The reactor was then connected to a gas reservoir composed of a mixture of CO and H₂ in equal partial pressures, and the total pressure in the reactor was adjusted to 30 bar. The temperature and pressure were held constant for 2 hours, whereafter the reactor was cooled and depressurised. The catalyst was filtered off and the contents were analysed by GC-MS and the conversion and n/iso ratio were calculated from these data.

**Table 3**

| Example No. | I-Rh | Heptenes | 1-octanal | 2-methylheptanal | 2-ethylhexanal |
|---|---|---|---|---|---|
| 1 | 0.50 g | 38% | 16% | 20% | 14% |

## Claims

1. Material having the general chemical formula 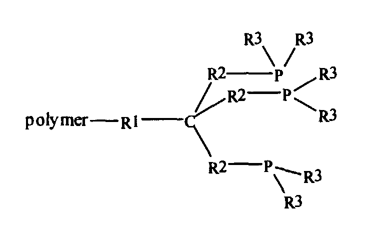 where R¹ is a linker and C(R²P(R³)₂)₃ group is a triphos moiety; and
wherein
R¹ is selected from branched and linear alkyl group and alkyl carbonyl group optionally substituted with functional groups comprising carbonyl, alkoxy, hydroxy, amino, nitro, carboxylic or sulfonic acid and esters or amides thereof;
R² are methylene (CH₂) or substituted methylene groups;
R³ are linear or branched alkyl or phenyl or substituted phenyl groups;
polymer is any polymer containing aromatic groups, comprising polystyrene, poly-vinyl pyridine, poly pyrrole, poly pyrrolidone or derivatives thereof.

2. The material of claim 1, wherein the polymer is substituted with one or more alkyl, formyl, alkyl carbonyl, alkoxy, hydroxy, amino, nitro or carboxylic or suphonic acid and esters or amides thereof in the aromatic ring.

3. The material of claim 1, wherein the polymer is a copolymer.

4. The material of claim 2, wherein the copolymer comprises a grafted polymer or block-copolymer.

5. The material of claim 1 being impregnated with transition metal salts or with carbonyl, organo metallic or other compounds of transition metals.

6. Use of a material according to anyone of the preceding claims as catalyst.
